# EUROPEAN PATENT SPECIFICATION

(11) **EP 3 275 436 B1**
(45) Date of publication and mention of the grant of the patent: **26.05.2021**
(21) Application number: 16773549.7
(22) Date of filing: 10.03.2016
(51) Int. Cl.: A61K 31/00, A23L 21/20, A61K 31/568, A61K 35/64, A61P 5/26

(54) **INSECT PROTEIN-CONTAINING PREPARATION FOR USE IN THE TREATMENT OF ANDROGEN DEFICIENCY IN WOMEN**
INSEKTENPROTEINHALTIGE ZUBEREITUNG ZUR VERWENDUNG IN DER BEHANDLUNG VON ANDROGENMANGEL BEI FRAUEN
PRÉPARATION DE PROTÉINES ENTOMOLOGIQUES UTILISÉE POUR TRAITER LE MANQUE D'ANDROGÈNES CHEZ LA FEMME

(30) Priority: 27.03.2015 RU 2015111086
(43) Date of publication of application: 31.01.2018
(73) Proprietor: Obshestvo S Ogranichennoj Otvetstvennostju "PARAFARM", Penza 440026 (RU)
(72) Inventor: STRUKOV, Villorij Ivanovich, Penza 440011 (RU); TRIFONOV, Vjacheslav Nikolaevich, Penzenskaya obl. 442960 (RU); ELISTRATOVA, Julia Anatolievna, Penza 440026 (RU); ELISTRATOV, Konstantin Gennadievich, Penza 440061 (RU); KURUS', Natal'a Vjacheslavovna, Penza 440026 (RU); FEDOROV, Alexandr Viktorovich, Penzenskaya obl. 442530 (RU); KRUTYAKOV, Evgeny Nikolaevich, Penza 440071 (RU); ANDREEVA, Elena Stanislavovna, Penza 440028 (RU); ASTAFJEVA, Alla Nikolaevna, Penza 440026 (RU); KUP OVA, Tat'jana Anatol'evna, Penza 440026 (RU); SHHERBAKOVA, Julija Gennad'evna, Penza 440018 (RU); SMIRNOVA, Natal'ja Mihajlovna, Penza 440003 (RU); ELISTRATOV, Georgiy Maksimovich, St.Petersburg 198324 (RU)
(74) Representative: Engel, Christoph Klaus
(86) International application number: PCT/RU2016/000130
(87) International publication number: WO 2016/159827

(56) References cited:
- EP-A1- 2 407 035
- EP-A1- 2 508 083
- EP-A1- 2 705 762
- EP-A1- 2 845 598
- WO-A1-2013/039424
- EA-B1- 014 932
- RU-C1- 2 412 616
- RU-C1- 2 425 593

## Description

The present invention relates to biochemistry, and specifically to entomologic proteins obtained by homogenization of drone brood which are used for normalizing of hormonal status and perceived general well-being in women. Specifically, the invention relates to a composition for use in the treatment of androgen insufficiency in women characterized in that it comprises drone brood homogenate.

Donators of androgens for women are obtained by homogenization of drone brood. Drone brood is a mixture of developing larvae, pre-pupae and pupae of male bees. Homogenization is made by homogenizers. Thus, obtained is homogenous mixture of proteins, including hormones and enzymes, fats, carbohydrates, oligonucleotides, nucleic acids and amino acids, vitamins, and macro- and microelements.

Each woman of reproductive age needs three sexual hormones (estrogens, progestins, and androgens) for normal life. Two of these hormones (estrogens and androgens) are especially necessary for normal course of aging process in women of post-menopausal age. One of the main causes of climax was considered insufficiency of estrogens that initiates climax processes regardless of causes of failure of ovarian function. Therefore, to decrease climacteric disorders is widely used estrogen replacement therapy. However, a number of researchers argue that androgens have greater importance in postmenopausal disorders. In support of this opinion must be noted the fact that androgenic receptors are present eventually in all organs and tissues of female organism such as bony tissue, central nervous system, skin, blood vessels, fat tissue, smooth and striated muscles.

Androgen insufficiency in women results in decrease of libido, depressed mood, decrease of muscle mass, decrease of bone mineral density, and decrease of perceived well-being. Adequate level of androgens can play vital role in metabolic, psychological, and sexual function in women.

Until the present time, the role of androgens in female organism is not completely investigated. It is established fact that androgens are necessary not only for development of reproductive function but also for maintaining of normal hormonal status in various age periods since androgens are hormones - precursors of estrogen biosynthesis in women. Researched are genomic and extra-genomic effects of androgens. The most peripheral organs and tissues such as bony tissue, mammary glands, sebaceous glands and hair follicles, skeletal muscles, fat tissue, and genitals have specific androgen receptors. Thereby, androgens have various effects on bone mineral density, muscle mass and strength, distribution of fat tissue, mood, sexual desire, cognitive function, and perceived general well-being. Besides of genomic mechanism, androgens can have extra-genomic effects, for example, on organs and tissues that have such enzyme as aromatase. These effects are provided by conversion of androgens to estrogens. The normal blood testosterone level in men is from 300 to 1000 ng/dl; in women the level of testosterone, according to various authors, is from 20-50 ng/dl to 10-120 ng\dl, i.e., about 10 times lower than in men [1-8]. Since the biologically normal level of androgens in women is significantly lower than in men, even some little disorder of androgenic formation of metabolism may lead to insufficiency of androgens in women.

The 1st International Conference on female androgen insufficiency (Princeton, USA, 2001) gave the definition of the "androgen insufficiency" (Al) as a new diagnostic term characterizing the above disorder. The term "androgens" is applied to C-19 steroids that are produced both in men and women in gonads and adrenal glands. They include testosterone (T), dehydroepiandrosterone (DHAE), dehydroepiandrosterone sulfate (DHAES), androstenedione (A) and 5α-dihydrotestosterone (DHT). As regards "A-androgens" (delta-androgens) - DHEA and DHEAS, 70-80% of their total amount is produced by adrenal glands, the most of them then are converted to A, T, DHT, and estrogens. The androgenic steroids T and DHT are biologically the most active. So, the daily production of testosterone (T) in organism of young healthy women is about 300 µg, i.e. 50% from their production in men. 98% of these hormones are bound with such protein as globulin which binds sexual steroids.

Depending on the severity of disorder, the following causes of Al in women are possible:
1. Ovariectomy, chemotherapy, or radiotherapy. So, ovariectomy leads to sharp decline of estrogenic and androgenic levels in the first 24 hours after surgery.
2. Adrenal insufficiency or adrenalectomy.
3. Pituitary cause (hypopituitarism).
4. Medication cause (intake of corticosteroids, antiandrogenic medicaments, or oral medicaments for hormone replacement therapy).
5. Idiopathic cause.

With the onset of menopause, testosterone (T) production gradually decreases. Decrease of the production of androgens begins as early as after the age of 30, and to the age of 70 is about 20% of its level in young age.

Diagnostics of Al includes collecting of psychosocial and medical histories, objective examinations and laboratory tests. The diagnosis of Al can be made only if estrogen level is normal. It applies to women of reproductive age with a normal menstrual cycle as well as to women of postmenopausal age who receive hormone replacement therapy (HRT). Detection of low androgen levels is an additional criterion for diagnosis of Al. However, this last sign as such cannot be considered as a basis for diagnosis of Al in absence of clinical signs. According to the Princeton Consensus statement on definition, classification and assessment of female androgen insufficiency (2001), blood serum level of testosterone (T) determined in the morning is recognized as the most reliable sign of the Al. This criterion is a "golden standard" in diagnostics of the androgen insufficiency (Al) in women. Thus, the diagnostics of Al is quite complex. Firstly, a physician must be convinced that the symptoms characteristic for Al are not resulted from estrogen insufficiency. When the estrogen level is normal, so characteristic symptoms of Al can be associated with one or more other somatic, endocrine disease such as hypo- or hyperthyroidism, metabolic syndrome, vitamin D insufficiency, chronic fatigue syndrome; chronic psychosocial stress at work and in the family, etc. Besides of low estrogen levels, the low T level may also be a cause of depression and decrease of perceived well-being in women [1-8].

When diagnosing Al must be excluded iatrogenic causes such as intake of antidepressants, serotonin reuptake inhibitors, sedative medicaments (barbiturates and benzodiazepines), antihypertensive medicaments from the group of β-adrenoblockers (propranolol, atenolol, etc.), calcium channel blockers (felodipine, israpidin), diuretics (spinolactone), hormonal medicaments (estrogens, progestogens, anti-androgens, corticosteroids). Prolonged intake of corticoids during treatment of rheumatoid arthritis or systemic lupus erythematosus, etc., also contributes to onset of Al. Alcohol and narcotic drugs also inhibit production of testosterone (T).

Mechanisms of androgenic effect of the above mentioned causes may be induced by decrease of androstenedione (A) and T production in ovaries, by competitive inhibition of 5α-reductase - enzyme that provides conversion of T to DHT in skin, and by decease of DHAES production. The most potent androgen are cyproterone acetate, dienogest, and chloramidinone acetate.

Treatment of Al is not yet developed. In Russia as well as in most countries of the world, there are no officially approved medicaments for androgen replacement therapy in women with Al. So, treatment of such patients is associated with certain difficulties. The presence of "residual" derivatives of 19-norsteroids having slight androgenic effect is used in combination with HRT medicaments in the treatment of women with Al in the perimenopausal (Climonorm, Trisequence) and postmenopausal age (Kliogest, Tibolone). If patients with postovariectomy syndrome, even with uterus removal, have expressed sexual disorders, severe astheno-depressive syndrome, it is preferable to use combined medicaments. Properly chosen HRT has combined beneficial effect on psycho-vegetative disorders and metabolic processes, prevents changes in proportions of the body, improves social adaptation and general life quality. However, wide use of HRT, especially in old age, is limited by a number of contraindications such as liver function disorders, tendency to thrombosis and thrombophlebitis, hyperplastic processes in uterus and mammal glands. Moreover, HRT increases the risk of breast cancer by 30-50%.

Currently are also used testosterone medicaments in form of injections, implants, patches and gels. However, all these medicaments are unsafe in the therapy of Al in women. Often there are side effects such as obesity, acne, growth of body and face hair, weight increase, voice deepening, anger spurts, and aggressiveness.

The prior art does not disclose recommendations for treatment of Al which would be based on results of wide randomized placebo-controlled clinical trials.

The main androgens in blood serum in women with normal menstrual cycle are testosterone and dihydrotestosterone. Dehydroepiandrosterone (DHAE), dehydroepiandrosterone sulfate (DHAES), and androstenedione are considered as prohormones since they can manifest their androgenic effects only when converted to testosterone. The organism of a healthy woman of reproductive age produces 300 µg of testosterone daily (i.e., 5% from its daily production in men). With aging women experience significant decrease of the levels of all androgens. So, the average levels of total and free testosterone, androstenedione, and DHAE in women in the age of 45 is 50%, in the age of 60 - about 30%, in the age of 70 - 10% from the levels of these hormones in 20-years-old women [4]. On the basis of the above, Kalinchenko S. et al. [5] believe that research of the role of androgens in women has great significance.

In the world practice there is a huge experience of the use of estrogen replacement therapy. However, there is increased evidence that often is not possible to improve the life quality without correction of age-related androgen insufficiency. The wide use of HRT, especially in old age, is limited by a number of contraindications such as liver function disorders, tendency to thrombosis and thrombophlebitis, hyperplastic processes in uterus and mammal glands. Moreover, HRT increases the risk of breast cancer by 30-50%.

In connection with the above, the search of new methods of treatment of the age-related Al is extremely important. Since the use of natural hormones - estrogens and androgens - has a number of disadvantages, it was suggested to use vegetable hormones. So, produced in USA "Citracal plus Vitamin D" contains genistein - soya hormone (analogue of female sexual hormones) - as substrate for synthesis of endogenous hormones. Authors of the claimed invention developed new technologies in treatment of Al with use of drone brood as donator of sexual hormones, and namely of testosterone, in women.

From the prior art is known a composition comprising drone brood and Potentilla alba (RU2425593, 10.08.2011). Based on this patent, the effect of drone brood on male organism is well investigated. The effect of drone brood on male organism was explained by stimulating of testicular function to produce endogenous testosterone.

Therefore, the use of drone brood for stimulating of testosterone production in female organism does not seem obvious, especially with regard to women in postmenopausal age because it was not clear what organ of female organism was to be stimulated to produce testosterone.

It was known from the prior art that androgens are produced in women either by adrenal cortex or by ovaries. As for women in menopausal age, the second way of testosterone synthesis can be excluded due to the overall decrease of ovarian function. As for the first way of androgen synthesis, adrenal glands produce androstenedione, dehydroepiandrosterone (DHAE), and 11-β-hydroxyandrostenedione which, as known, are precursors of testosterone (Filippovich Yu.B. Biochemical basis of human life. Moscow: Vlados, 2005. P.336).

Authors of the invention assumed that the production of testosterone in female organism is contributed by liver that disintegrate entomologic testosterone to the level of human testosterone.

But whatever may be the way of testosterone synthesis in female organism, it is not possible to draw parallels with synthesis ways of this hormone in male organism. So, as known, all steroids are lipid compounds which are poorly soluble in water. Therefore they are present in blood mainly in bound state with blood plasma proteins. Only a small portion of these compounds is in free form. It is just free steroids have high biological activity: they penetrate the membrane of target cells. Precursor of steroids is cholesterol that converts to progesterone in the cells of adrenal cortex. Progesterone, in turn, may be converted to corticoids, androgens, and estrogens (Liubimova Z.V. Age physiology. Part 1. Moscow: Vlados, 2004. P.105). One of dietary recommendations for increase of testosterone level in men includes usually increase of consumed cholesterol, for example, eggs. However, this recommendation is unacceptable for women because it may lead to increase of estrogens or corticoids levels. It is known from the prior art that the same stimuli to the internal secretion glands of men and women have different effects on hormone synthesis. So, follicle-stimulating hormone (FSH) secreted by pituitary gland results in production of estrogens in women and production of testosterone in men, respectively (Gurovets G.V. Age anatomy and physiology. Moscow: Vlados, 2013. P.215). Thus, despite the fact that the effect of drone brood as stimulator of testosterone productions in men is known from the prior art, it is necessary to take into account the differences of endocrine system functioning in men and in women. Therefore, it was not obvious to expect that substances which influence the central links controlling testosterone synthesis in men (Krylov, V. ; Krivtsov, A.; Lebedev, N.; Burimistrova, V.; Oshevenski, L.; Sokolski, L.: Theory and agents of apitherapy; Publisher: Comilfo (Russia); ISBN : 978-5-903535-03-3) would have the same effect on female organism. Moreover, it could not expect that the same substances which regulate secretion of testosterone by testicles in men would stimulate secretion of testosterone in women.

The present invention is based on unexpected discovery of the fact that drone brood has androgenotropic effect on female organism.

The technical result of the claimed invention consists in increase of level of endogenous androgens in blood in women, particularly, of testosterone.

Earlier, the authors of the claimed invention reported that drone brood is donator of various kinds of sexual hormones (estradiol, progesterone, and testosterone) both in men and in women (RU2498811, 20.11.2013; RU2412616, 27.02.2011). However, at that time it was not known donator of which sexual hormones for women is drone brood. I.e., the cited publications did not give any data which would disclose that drone brood could increase the blood testosterone level in women. Based on the prior art, it would be reasonable to expect that it rather will lead to increase of estradiol or progesterone level in women. Although various sources mention pronounced gonadotropic effect that stimulates gonads' activity, it is known that drone brood homogenate is stimulator of central regulatory mechanisms of secretion of male sexual hormones (androgens) that also restores biochemical characteristics of testicles (concentration of testosterone in blood and that of fructose in seminal vesicle fluid), prostate gland, and sperm production. The use of drone brood is effective against sexual dysfunction in men, male infertility, and decrease of sexual desire. The intake of drone brood homogenate stimulates ovarian function in women, restores hormonal status and reproductive function. Thus, it was known from the prior art that drone brood homogenate stimulates androgen production in men and estrogen production in women.

The cited publications refer to decisions related to the treatment of osteoporosis. It is known from the prior art that osteoporosis in women may have estradiol-dependent nature. Summing up the above, the authors conclude that analysis of the prior art did not disclose any decision which would lead to obtaining of the claimed technical result - increase of the level of endogenous androgens in blood in women, particularly, of testosterone, - when using biologically active substances, particularly, drone brood.

It has been found that the effect of entomologic testosterone significantly differs from that of exogenous, for example, from the effect of testosterone propionate, the use of which is detrimental to female health (masculinization, acne, voice deepening, excessive growth of body hair, male-pattern baldness, enlargement of the clitoris, and thrombophlebitis.

Given the fact that the liver converts testosterone propionate to estrogens, it would not be logical to assume that entomologic testosterone will be converted by the liver to the human testosterone.

The closest analogue of the claimed invention is the decision disclosed in EA14932, 29.04.2011. The above decision discloses method of treatment of the conditions associated with decrease of androgen level in women, comprising the use of selective androgen receptor modulators.

The authors created the composition based on drone brood homogenate made in form of powder, tablets or capsules.

The authors conducted a study to research effects of the created composition on hormonal status of women with androgen insufficiency.

Materials and methods. From 2009 to March 2013 examined were 72 women in the age from 49 to 85. Criteria for inclusion in the study were following: women with hormonally and clinically confirmed androgen insufficiency.

The research included objective examination, general clinical laboratory tests as well as hormonal tests - determination of the total testosterone, globulin, and sex hormone-binding globulin (SHBG) levels. Hormonal tests were carried out by immunochemiluminescent method on the "Immulite 2000" device.

All tested women were divided into 2 comparative groups depending on degree and severity of the disease: 1st group (38 women) received the claimed composition orally - one tablet in the morning and at night (1 tablet contained 100 mg of drone brood) for 3 months 3 times a year. 2nd (control) group (34 women) received placebo - two times per day by the same regimen as in the 1st group.

Statistical analysis of the received data was performed using software package StatSoft, Windows XP. Quantitative characteristics were described by mean and standard deviations. The data are presented on the format: M±m where M is arithmetic mean, and m is standard deviation. Differences were considered as statistically significant at significance level p<0.05.

### Results.

After the end of the trial, in the group of women who received the claimed composition, there was observed mood improvement, increase of activity, increase of muscle strength, decrease of dysuretic disorders and of osteoporotic activity.

In the control group of women who received placebo, there was not observed any effects that were present in the 1st group.

Analysis of hormonal characteristics revealed that the total testosterone level in women from both tested groups before treatment was:
1st group: 1.1±0.4 nmol/l;
2nd group 1.2±0.5 nmol/l (p<0.05),
with reference values for this method 1.7-3.4 nmol/l. Levels of sex hormone-binding globulin (SHBG) in the 1st and 2nd groups before treatment were 64.3±2.6 nmol/l and 62.8±2.9 nmol/l (p<0.05), respectively. After 9-months treatment with the claimed composition, 29 patients from the group of 37 women reported improvement. When assessing laboratory characteristics, there was noted increase of the levels of total testosterone in blood serum from 1.1±0.4 nmol/l to 2.5±0.6 nmol/l (p<0.05), SHBG from 64.3±2.6 to 115.0±5.9 nmol/l (p<0.05). The 2nd group which received placebo no one of patients had any positive changes in testosterone level and increase of SHBG.

The results of this research clearly demonstrate that the intake of the claimed composition normalizes androgen level in women. It leads to improvement of general well-being, disappearance or reduction of the clinical symptoms of Al. This effect may be obtained when taking the claimed composition in amount of 10 mg to 600 mg daily.

The received data show that aging process is accompanied by decrease of hormonal androgen status that leads to the appearance of the clinical symptoms of Al.

### Literature:

1. Marchenko L.A., Ilyina L.M. Informational material on androgen insufficiency for distribution to members of Association of gynecologists-endocrinologists. 2005.
2. Kalinchenko S.Yu., Apetov S.S. Role of androgens in women: what we know? // Attending doctor 2010; 8: 78-83.
3. Kalinchenko S.Yu., Apetov S.S. Use of androgens in women in the menopausal age // Attending doctor 2009; 3: 28-30.
4. Riggs B.L., Hodgson I.F., O'Fallon W.M. et al. Fluoride treatment on the fracture rate in postmenopausal women with osteoporosis // New England Journal of Medicine, 1990; 322: 802-809.
5. Kalinchenko S.Yu., Vishnevskiy E.L., Koval A.N., Mskhalaya G.J., Saad F. Benefitial effects of testosterone administration on symptoms of the lower urinary tract in men with late-onset hypogonadism: A pilot study//The Aging Male. 2008, vol.11, Iss.2: 57-61.
6. Mudali S., Dobs A.S., Ding J., Cauley J.A., Szklo M., Golden S.H. Endogenous postmenopausal hormones and serum lipids: the Atherosclerosis Risk in Communities Study // The Journal of Clinical Endocrinology and Metabolism, 2005, vol.90, p.1202-1209.
7. Nathorst-Boos J., Floter A., Jarcander-Rollf M. Treatment with percutaneous testosterone gel in postmenopausal women with decreased libido-effects on sexuality and psychological well-being // Maturitas, 2006, vol.53, p.11-18.
8. Riverra-Woll L.M., Papalia M., Davis S.R., Burger H.G. Androgen insufficiency in women: diagnostic and therapeutic implications // Human Reproduction Update, 2004, vol.10, Nr.5, p.421-432.

## Claims

1. A composition for use in the treatment of androgen insufficiency in women **characterized in that** it comprises drone brood homogenate.

2. The composition according to claim 1 for use in the treatment of androgen insufficiency in women where the composition is made in the form of tablets, capsules or powder.

## Patentansprüche

1. Zusammensetzung zur Verwendung bei der Behandlung von Androgen-Insuffizienz bei Frauen, **dadurch gekennzeichnet, dass** sie Drohnenbrut-Homogenat umfasst.

2. Zusammensetzung nach Anspruch 1 zur Verwendung bei der Behandlung von Androgen-Insuffizienz bei Frauen, wobei die Zusammensetzung in Form von Tabletten, Kapseln oder Pulver hergestellt ist.

## Revendications

1. Composition destinée à être utilisée dans le traitement de l'insuffisance d'androgène chez les femmes **caractérisée en ce qu'**elle comprend un homogénat de couvain à faux bourdons.

2. Composition selon la revendication 1 destinée à être utilisée dans le traitement de l'insuffisance d'androgène chez les femmes où la composition est constituée sous la forme de comprimés, de capsules ou de poudre.
